# EUROPEAN PATENT SPECIFICATION

(11) **EP 3 185 779 B1**
(45) Date of publication and mention of the grant of the patent: **21.12.2022**
(21) Application number: 15767297.3
(22) Date of filing: 10.08.2015
(51) Int. Cl.: A61B 8/08

(54) **CONCURRENT ACQUISITION OF HARMONIC AND FUNDAMENTAL IMAGES FOR SCREENING APPLICATIONS**
GLEICHZEITIGE ERFASSUNG VON HARMONISCHEN UND GRUNDLEGENDEN BILDERN FÜR SCREENING-ANWENDUNGEN
ACQUISITION SIMULTANÉE D'IMAGES HARMONIQUES ET FONDAMENTALES POUR DES APPLICATIONS DE DÉPISTAGE

(30) Priority: 28.08.2014 US 201462042980 P
(43) Date of publication of application: 05.07.2017
(73) Proprietor: Koninklijke Philips N.V., 5656 AG Eindhoven (NL)
(72) Inventor: JAGO, James Robertson, NL-5656 AE Eindhoven (NL)
(74) Representative: Philips Intellectual Property & Standards
(86) International application number: PCT/IB2015/056064
(87) International publication number: WO 2016/030785

(56) References cited:
- WO-A1-2013/183651
- US-A- 5 873 829
- US-A1- 2003 013 959
- US-A1- 2003 055 337
- US-A1- 2007 010 747
- US-A1- 2007 161 898
- US-A1- 2008 119 733

## Description

### BACKGROUND:

### Technical Field

This disclosure relates to medical instruments and more particularly to diagnostic ultrasound where different imaging modes (e.g., fundamental and harmonic images) are acquired during a single screening acquisition, so that any mode can later be selectively reviewed.

### Description of the Related Art

Breast density is one of the strongest predictors of a failure of mammography to detect cancer and is also a well-established predictor of breast cancer risk. A basic concept of breast ultrasound screening is that an operator, who may or may not be skilled in interpreting ultrasound images, acquires ultrasound images covering all of the breast tissue as efficiently as possible. The acquisition may be performed free-hand or free-hand with electromagnetic (EM) tracking. The acquisition may also be semi-automated or fully automated. In all of these cases, the reading and interpretation of these images is performed very efficiently off-line by a radiologist. The radiologist reviews a complete set of images on a workstation and may also review images that are rendered from the images collected, such as "C" plane images.

Patients with suspicious findings are called back for diagnostic ultrasound. Since the radiologist is making important decisions about the need to call back the patient, with the associated costs and patient anxiety, it is important that the radiologist has as much information as possible to decide if a lesion is truly suspicious, and not just a simple cyst, for example.

In conventional diagnostic ultrasound, where a suspicious lesion is being characterized, it is typical clinical practice to use many different ultrasound imaging modes to decide whether a lesion has the characteristics of cancer or is more likely benign. For example, fundamental imaging is typically best for visualizing the internal structure of a solid lesion, whereas harmonics may be best for identifying cysts since clutter artifacts are reduced. Spatial compounding (e.g., SonoCT) is best for visualizing the borders of a lesion, the irregularity of which is an important indicator for malignancy. Spatial compounding has the downside of suppressing shadowing, which is itself an important indicator.

One of the limitations with existing breast ultrasound screening solutions, regardless of whether the images are acquired manually or with automation, is that the operator must decide on the best ultrasound imaging mode to use for acquisition, e.g., either fundamental or harmonic, spatial compounding or no compounding, color, color power angioplasty, elastography, etc. This is because there is insufficient time to re-acquire sweeps in every mode, and there is insufficient time for a radiologist reading the exam to review all the data sets individually. In practice, screening is typically performed in fundamental mode since, in harmonic mode, shadows from lesions are stronger and may also be caused by multiple other targets such as Cooper's Ligaments. These shadows make the subsequent reading of the images much harder for the radiologist. For example, strong shadows can look like hypo-echoic lesions in "C" plane rendered images, and shadows from Copper's Ligaments can be very distracting. Thus, in a typical screening workflow, the radiologist is limited to the fundamental images (with or without spatial compounding), and is not able to use harmonics or enable/disable compounding to assist in deciding whether a lesion is suspicious.

### SUMMARY

Each of the documents WO2013/183651 and US5873829 discloses a method and a system for acquiring ultrasound frames of multiple modes in a single acquisition scan.

The invention is defined by the independent claims. Advantageous embodiments are provided in the dependent claims.

A method for providing multiple review modes in a single acquisition scan is disclosed in claim 1.

A system for providing multiple review modes in a single acquisition scan is disclosed in claim 7.

These and other objects, features and advantages of the present disclosure will become apparent from the following detailed description of illustrative embodiments thereof, which is to be read in connection with the accompanying drawings.

### BRIEF DESCRIPTION OF DRAWINGS

This disclosure will present in detail the following description of preferred embodiments with reference to the following figures wherein:
FIG. 1 is a block/flow diagram showing an ultrasonic system configured to collect multiple imaging modes in a single acquisition sequence in accordance with one embodiment;
FIG. 2A is a diagram showing an alternating mode pattern for acquiring images for two imaging modes in accordance with one embodiment;
FIG. 2B is a diagram showing an alternating mode pattern for acquiring images where images are taken with a set number of sequential frames for one or more imaging modes in accordance with other embodiments;
FIG. 3 is a block/flow diagram showing a review workstation for reviewing stored ultrasonic images from a single acquisition sequence in accordance with one embodiment; and
FIG. 4 is a block/flow diagram showing methods for providing multiple review modes in a single acquisition scan in accordance with illustrative embodiments.

### DETAILED DESCRIPTION OF EMBODIMENTS

In accordance with the present principles, ultrasound screening methods and systems are provided where ultrasound data acquired during a single scan can be employed for generating multiple imaging modes, e.g., fundamental imaging, harmonic imaging, color imaging, color power angioplasty imaging, elastography imaging, etc. Conventional scan and review processes often employ an automated scanning of tissue where the scan mode is typically for a single imaging mode. In accordance with the present principles, a single scan sequentially provides frames for a plurality of different imaging modes. The frames are stored and can be employed later to generate a display for review for each of the plurality of imaging modes. In other words, as a result of a single scan, fundamental images, harmonic images, etc. may be reviewed without the requirement for multiple particular mode scans (e.g., fundamental, harmonic, fundamental with compounding, etc.). In diagnostic ultrasound (e.g., real-time scanning), it is common to use both fundamental and harmonic imaging to characterize a lesion, since each provides some unique information. During diagnostic ultrasound, different imaging modes may be employed. However, for screening with stored ultrasound images, the mode was previously selected during the scan, and employing different imaging modes was not available for a reviewer. In accordance with the present principles, a plurality of imaging modes are collected including fundamental and harmonic images during screening acquisition, so that the clinician can look at either mode later in review. This may be achieved by collecting sufficiently raw data and applying software post-processing to generate the desired modes this way does not fall under the scope of the present invention, or during acquisition, automatically switching between the modes for a set duration to acquire data for multiple modes.

It should be understood that the present invention will be described in terms of medical instruments; however, the teachings of the present invention are much broader and are applicable to any ultrasonic imaging system and method. In some embodiments, the present principles are employed in tracking or analyzing complex biological or mechanical systems. In particular, the present principles are applicable to internal tracking procedures of biological systems, procedures in all areas of the body such as the breasts, lungs, gastro-intestinal tract, excretory organs, blood vessels, liver, etc. The elements depicted in the FIGS. may be implemented in various combinations of hardware and software and provide functions which may be combined in a single element or multiple elements.

The functions of the various elements shown in the FIGS. can be provided through the use of dedicated hardware as well as hardware capable of executing software in association with appropriate software. When provided by a processor, the functions can be provided by a single dedicated processor, by a single shared processor, or by a plurality of individual processors, some of which can be shared. Moreover, explicit use of the term "processor" or "controller" should not be construed to refer exclusively to hardware capable of executing software, and can implicitly include, without limitation, digital signal processor ("DSP") hardware, read-only memory ("ROM") for storing software, random access memory ("RAM"), non-volatile storage, etc.

Moreover, all statements herein reciting principles, aspects, and embodiments of the invention, as well as specific examples thereof, are intended to encompass both structural and functional equivalents thereof. Additionally, it is intended that such equivalents include both currently known equivalents as well as equivalents developed in the future (i.e., any elements developed that perform the same function, regardless of structure). Thus, for example, it will be appreciated by those skilled in the art that the block diagrams presented herein represent conceptual views of illustrative system components and/or circuitry embodying the principles of the invention. Similarly, it will be appreciated that any flow charts, flow diagrams and the like represent various processes which may be substantially represented in computer readable storage media and so executed by a computer or processor, whether or not such computer or processor is explicitly shown.

Furthermore, embodiments of the present invention can take the form of a computer program product accessible from a computer-usable or computer-readable storage medium providing program code for use by or in connection with a computer or any instruction execution system. For the purposes of this description, a computer-usable or computer readable storage medium can be any apparatus that may include, store, communicate, propagate, or transport the program for use by or in connection with the instruction execution system, apparatus, or device. The medium can be an electronic, magnetic, optical, electromagnetic, infrared, or semiconductor system (or apparatus or device) or a propagation medium. Examples of a computer-readable medium include a semiconductor or solid state memory, magnetic tape, a removable computer diskette, a random access memory (RAM), a read-only memory (ROM), a rigid magnetic disk and an optical disk. Current examples of optical disks include compact disk - read only memory (CD-ROM), compact disk - read/write (CD-R/W), Blu-Ray^{™} and DVD.

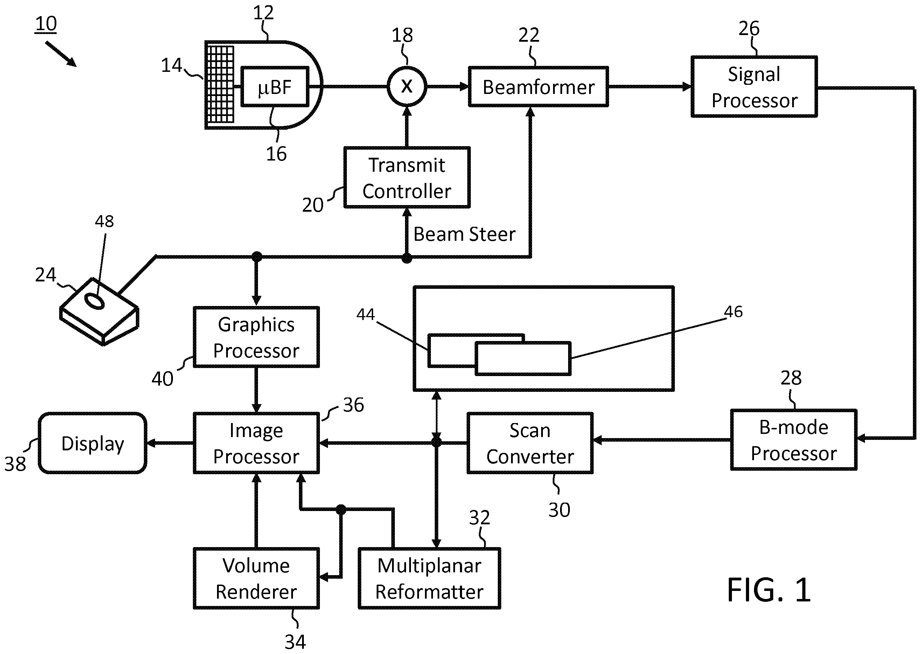

Referring now to the drawings in which like numerals represent the same or similar elements and initially to FIG. 1, an ultrasound imaging system 10 constructed in accordance with the present principles is shown in block diagram form. In the ultrasonic diagnostic imaging system of FIG. 1, an ultrasound system 10 includes a probe 12 having a transducer or transducer array 14 for transmitting ultrasonic waves and receiving echo information. A variety of transducer arrays are well known in the art, e.g., linear arrays, convex arrays or phased arrays. The transducer array 14, for example, can include a two dimensional array (as shown) of transducer elements capable of scanning in both elevation and azimuth dimensions for 2D and/or 3D imaging. The transducer array 14 is coupled to a microbeamformer 16 in the probe 12, which controls transmission and reception of signals by the transducer elements in the array. In this example, the microbeamformer 16 is coupled by the probe cable to a transmit/receive (T/R) switch 18, which switches between transmission and reception and protects a main beamformer 22 from high energy transmit signals. In some embodiments, the T/R switch 18 and other elements in the system can be included in the transducer probe rather than in a separate ultrasound system base. The transmission of ultrasonic beams from the transducer array 14 under control of the microbeamformer 16 is directed by a transmit controller 20 coupled to the T/R switch 18 and the beamformer 22, which may receive input from the user's operation of a user interface or control panel 24.

In accordance with one embodiment, the transmit controller 20 automatically switches the imaging modes for one or more frames to concurrently acquire (receive) echoes/images in multiple imaging modes. A user may adjust the relative frame rate between the imaging modes using the interface 24, and, in particular, a frame rate control 48. Another function controlled by the transmit controller 20 is the direction in which beams are steered. Beams may be steered straight ahead from (orthogonal to) the transducer array, or at different angles for a wider field of view. The partially beamformed signals produced by the microbeamformer 16 are coupled to a main beamformer 22 where partially beamformed signals from individual patches of transducer elements are combined into a fully beamformed signal.

The beamformed signals are coupled to a signal processor 26. The signal processor 26 can process the received echo signals in various ways, such as bandpass filtering, decimation, I and Q component separation, and harmonic signal separation. The signal processor 26 is also configured to perform additional signal enhancement such as signal compounding and optionally speckle reduction and noise elimination. The processed signals are coupled to a B mode processor 28, which can employ amplitude detection for the imaging of structures in the body. The signals produced by the B mode processor are coupled to a scan converter 30 and a multiplanar reformatter 32. The scan converter 30 arranges the echo signals in the spatial relationship from which they were received in a desired image format. For instance, the scan converter 30 may arrange the echo signal into a two dimensional (2D) sector-shaped format, or a pyramidal three dimensional (3D) image. The multiplanar reformatter 32 can convert echoes which are received from points in a common plane in a volumetric region of the body into an ultrasonic image of that plane, as described in U.S. Pat. No. 6,443,896 (Detmer). A volume renderer 34 converts the echo signals of a 3D data set into a projected 3D image as viewed from a given reference point, e.g., as described in U.S. Pat. No. 6,530,885 (Entrekin et al.). The 2D or 3D images are coupled from the scan converter 30, multiplanar reformatter 32, and volume renderer 34 to an image processor 36 for further enhancement, buffering and temporary storage for display on an image display 38. A graphics processor 40 can generate graphic overlays for display with the ultrasound images. These graphic overlays or parameter blocks can contain, e.g., standard identifying information such as patient name, date and time of the image, imaging parameters, frame indices and the like. For these purposes, the graphics processor 40 receives input from the user interface 24, such as a typed patient name. The user interface 24 can also be coupled to the multiplanar reformatter 32 for selection and control of a display of multiple multiplanar reformatted (MPR) images.

In accordance with the present principles, screening ultrasound data is acquired and stored in memory 42 in a format that allows an offline reader/reviewer to still be able to access multiple imaging modes, e.g., to help characterize a suspicious lesion, etc., but without significantly complicating or extending the workflow for either acquisition or review. In one embodiment, the ultrasound system 10 is programmed to acquire images that alternate between modes, e.g., fundamental, harmonic, etc. In one example, to make acquisition easier for the operator, only the fundamental (or harmonic) images are shown on the display 38, the other images are acquired but not shown. The memory 42 stores frames in memory structures or logically connects (e.g., points to or indexes) frames of a same mode. This may include organizing frames of a stream by the use of indexing, multiplexing or other techniques to designate which frames are associated with each imaging mode. In one embodiment, the frames may be stored in separate data structures 44, 46 for easy access when attempting to regenerate an image in a particular imaging mode. The memory 42 is depicted as being placed after the scan converter 30; however, the memory 42 may store data at any position in the signal path. In particularly useful embodiments, not falling under the scope of the present invention, the data stored may be sufficiently raw data that needs to be stored earlier in the signal path to permit the raw data to be available for rendering in multiple modes. In such a case, the switching between modes is not needed as the data will be stored to recreate these modes in post-processing to generate the desired modes during or for review.

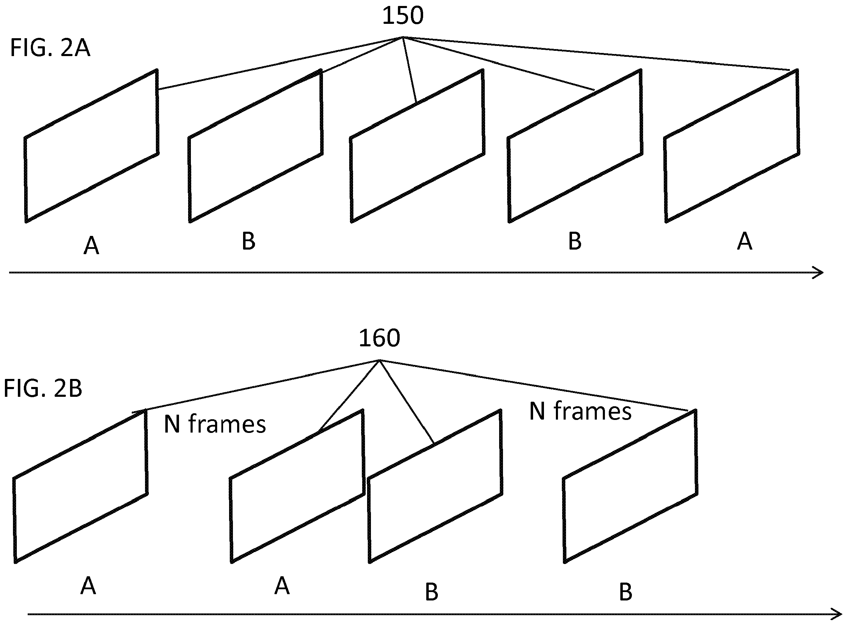

The resolution of one mode may be adjusted using a frame rate control 48. The frame rate control 48 may be employed to adjust the number of frames for one mode versus the other modes. The frame rate control 48 may be implemented in software, hardware or a combination of both. This will be further described with reference to FIGS. 2A and 2B.

Referring to FIGS. 2A and 2B, diagrams illustratively show imaging mode frame collection. FIG. 2A shows acquired frames 150, which alternate between a first mode (mode A) and a second mode (mode B). In one embodiment, mode A and mode B may include fundamental and harmonic imaging modes. For each mode, the frame rate will be reduced by a factor of 2, but since ultrasound systems are now able to acquire images at high frame rates that accuracy/resolution is not compromised. For example, frame rate settings (speed) are preferably over 100 Hz.

The imaging mode streams may be separated and both streams of, e.g., fundamental and harmonic imaging may be stored and exported as separate but linked loops. The linking may include the use of time stamps, frame numbers, indexes, etc. When a radiologist initially reviews the data, the radiologist may only see the fundamental images since these have the least artifactual shadowing and are thus most efficient for finding suspicious lesions. However, once a suspicious lesion has been identified the radiologist can switch to the equivalent harmonic image obtained from the same location, and thus obtain diagnostic information from both modes. Note that this description refers to screening the images, which is typically performed in a separate process than scanning. The scanned images are often done with a set imaging mode. In conventional systems, a reviewer is often stuck with the selected imaging mode. In accordance with the present principles, the same workflow provides access to multiple imaging modes. These imaging modes are compiled at a later time (not necessarily during scanning), and the reviewer can select the imaging mode to display with minimal loss of accuracy and no time lost during the scanning process. The screening process includes the review of the images at a separate time and/or location, e.g., at a workstation configured for reviewing.

FIG. 2B shows a more general frame collection scheme where one or multiple frames 160 are collected for each imaging mode. In one embodiment, a single mode A frame may be collected followed by N (an integer number) mode B frames, and then repeated. In another embodiment, N mode A frames may be taken followed by a single mode B frame and then repeated. In yet another embodiment, N mode A frames may be collected followed by N mode B frames, and then repeated. In other embodiments, a greater number of modes may be employed and different combinations of frame numbers may be employed. The additional frame numbers for a particular imaging mode may be selected to increase resolution for that imaging mode relative the other imaging mode or modes. These adjustments in frame numbers may be selected for the scanning based on experience, desired results or other criteria.

Spatial compound imaging or spatial compounding (SonoCT) is an ultrasound technique that uses electronic beam steering of a transducer array to rapidly acquire several (e.g., three to nine) overlapping scans of an object from different view angles. These single-angle scans are averaged to form a multiangle compound image that is updated with each subsequent scan. Compound imaging shows improved image quality compared with conventional ultrasound, primarily because of reduction of speckle, clutter, and other acoustic artifacts, and provides improved contrast resolution and tissue differentiation, which are beneficial for imaging the breast, peripheral blood vessels, and musculoskeletal injuries.

Spatial compounding being switched on or off can also be accommodated at the screening stage by a user in accordance with the present principles. Ultrasound data would be acquired in SonoCT (and if desired fundamental and harmonic modes), and the operator or scanner would see a SonoCT image, but the component frames and not the compounded frames would be stored in memory 42 (e.g., as a separate mode). During review, workstation software would perform the compounding step needed to generate a SonoCT image to present to the radiologist, or the radiologist could choose to view the non-SonoCT image (i.e., to asses lesion shadowing) in which case only the non-steered component image would be presented. The post-processing of the image stream may be performed with or without the mode switching process step - the later not being part of the invention.

For example, the switching mode data collection can be post-processed to switch compounding on or off. Likewise, the sufficiently raw data can be collected and be post-processed to switch compounding on or off.

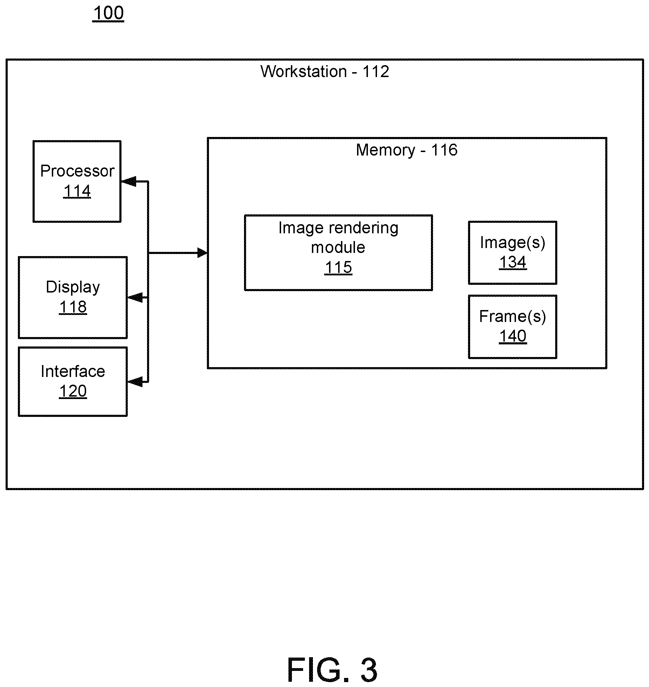

Referring to FIG. 3, a system 100 for review of ultrasound images is illustratively shown in accordance with one embodiment. System 100 may include a workstation or console 112 from which images are reviewed and modes selected. Workstation 112 preferably includes one or more processors 114 and memory 116 for storing programs, applications and data. Memory 116 may store an image rendering module 115 configured to collect and render image frames for the display of one or more imaging modes.

The image rendering module 115 is configured to receive image data and link or process image modes for display. An image 134 can be generated from frames 140 stored in memory 116 and can be displayed on a display device 118. Workstation 112 includes the display 118 for reviewing internal images of a subject (e.g., a patient). Display 118 may also permit a user to interact with the workstation 112 and its components and functions, or any other element within the system 100. This is further facilitated by an interface 120 which may include a keyboard, mouse, a joystick, a haptic device, or any other peripheral or control to permit user feedback from and interaction with the workstation 112.

In one embodiment, each imaging mode is acquired sequentially and so each mode can be fully optimized without compromise, for example, in terms of the acquisition design (e.g., line density, focal zones), signal and image processing, display parameters, etc. For example, for SonoCT (spatial compounding), the non-steered frame may be designated differently from the other component frames, since this non-steered frame will be visualized on its own without the benefit of compounding. That is, it may have a different line density, more focal zones, more frequency compounding, or a different display map.

In another embodiment, the review and interpretation of the images may be performed either on the system 10 (FIG. 1) or off the system on the workstation 100. In either case, review software of image rendering module 115 needs to be able to support the capability to correctly process the separate modes being presented. For fundamental versus harmonic imaging, this may only involve pulling images from the appropriate data stream and applying a suitable display map. For SonoCT versus non-SonoCT, the review software of image rendering module 115 needs to be able to extract only the non-steered frames and apply appropriate display maps for non-SonoCT, or extract all the frames and apply a compounding algorithm to them, plus appropriate display mapping for SonoCT.

In accordance with an alternate embodiment - not being part of the invention, instead of acquiring images by switching modes, the acquisition may be obtained as a sufficiently raw data stream. In such an embodiment, the acquisition (initial scan) is more efficient because all of the imaging acquired can be utilized to display in more than one mode (i.e., there are no alternating modes). For example, both the fundamental and harmonic images that are displayed in review may be extracted from the same acquisition and the same data. The modes extracted from the data may be based on software operations rather than mode switching. For example, a single kind of data acquisition is stored which includes all mode components, and these are separated through additional processing by image rendering module 115, such as, e.g., band-pass filtering of IQ data, applying different summation weights to radio frequency (RF) data acquired with opposite polarity transmit waveforms, etc. The stored data is stored in a format that is sufficiently raw (i.e., minimally processed) so that the components of all modes (e.g., fundamental and harmonic) can be extracted with sufficient quality.

For example, both the fundamental and harmonic images that are displayed in review may be extracted from the same acquisition. Only one kind of acquisition is defined and stored during scanning, which includes both fundamental and harmonic components (and other modes), and these are separated through additional processing at the workstation 112. The additional processing may be performed by the image rendering module 115 and may include functions such as image filtering, band-pass filtering of IQ data, applying different summation weights to RF data acquired with opposite polarity transmit waveforms, etc.

The present principles are particularly useful in medical procedures such as for breast screening or other screening procedures. The present principles may also be applicable to any clinical application that involves the need for rapid acquisition with minimal interpretation during acquisition, followed by careful review and interpretation post-acquisition. One example may include screening for liver cancer with ultrasound. Target platforms for the present principles include any ultrasound systems and workstations designed for screening purposes.

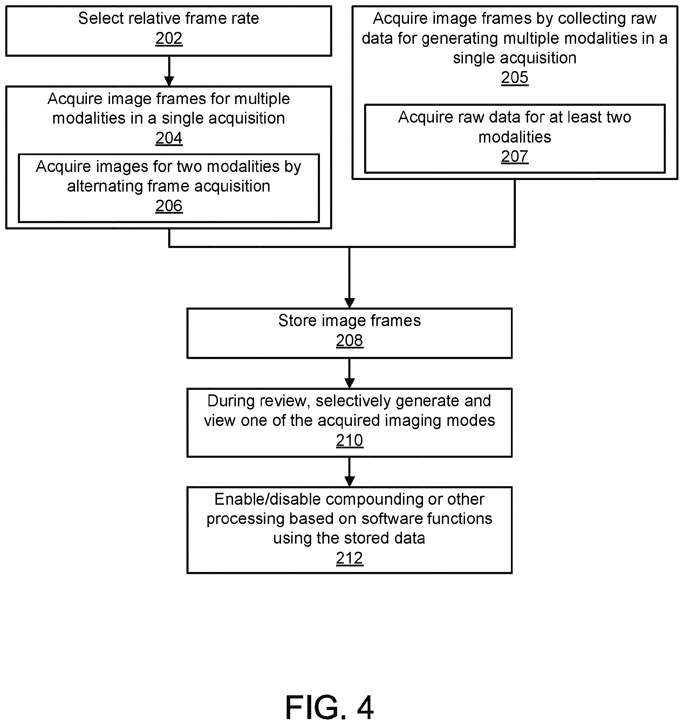

Referring to FIG. 4, methods for providing multiple review modes in a single acquisition scan are illustratively shown. In block 202, a relative frame rate may be selected for each of the plurality of imaging modes in the acquisition sequence. The frame rate may be programmed into the system (e.g., by controlling the transmitter) to toggle between different imaging modes for different consecutive durations to control the number of frames received for each imaging mode. The relative frame rate between imaging modes may include an integer multiple of successive frames for at least one of the imaging modes in the acquisition sequence.

In block 204, image frames are acquired. This may include acquiring image frames for a plurality of imaging modes by switching image acquisition modes during a single acquisition sequence. In block 206, the imaging frames may be acquired from at least two imaging modes (e.g., two) and the image acquisition modes are switched such that frames are acquired in an alternating manner for each of the two modes. Alternately, acquiring image frames includes acquiring data that is sufficiently raw to generate images in multiple modes by soft processing in block 205. This means that the collected data includes sufficient information for generating each of the desired imaging modalities. In one embodiment, raw data is acquired for generating at least two imaging modes in block 207.

The imaging modes include ultrasonic imaging modes, and the ultrasonic imaging modes include fundamental, harmonic, and optionally one or more of compound fundamental, compound harmonic, color, color power angioplasty, elastography, etc. During the acquisition, the image frames may be acquired by scan and displayed to an operator in a single imaging mode during the single acquisition.

In block 208, the image frames are stored in non-transitory memory for each acquisition mode for subsequent review. The storage may include storing images of a particular mode together (in a separate device, memory partition etc.), logically linking the images of a particular mode, employing indexing of images and providing a lookup table, generating the mode images from sufficiently raw data, etc.

In block 210, during review (e.g., post scan, not live), a display is selectively generated for each of the plurality of imaging modes from stored images for a selected imaging mode such that each of the plurality of image modes is available for review from the single acquisition sequence. The generation of images may include post-processing of the sufficiently raw data, provide grouping of collected frames from a single imaging mode, etc.

A reviewer can switch between different imaging modes, e.g., fundamental, harmonic, compound fundamental, compound harmonic, etc. to obtain a more accurate result by employing the strength of each imaging mode. Since the desired imaging modes are all available, there is a significantly reduced need to rescan the patient in other imaging modes as in conventional workflows. The plurality of imaging modes may include multiple imaging modes for discovering different diagnostic information employed for identifying a lesion in an organ, or other applications. Such applications may include breast cancer screening, liver cancer screening, etc.

In block 212, enabling/disabling compounding using the stored images may be performed using software functions. Other image processing functions may also be performed using software functions, e.g., filtering, contrast enhancement, generating modes from raw data, etc.

In interpreting the appended claims, it should be understood that:
a) the word "comprising" does not exclude the presence of other elements or acts than those listed in a given claim;
b) the word "a" or "an" preceding an element does not exclude the presence of a plurality of such elements;
c) any reference signs in the claims do not limit their scope;
d) several "means" may be represented by the same item or hardware or software implemented structure or function; and
e) no specific sequence of acts is intended to be required unless specifically indicated.

Having described preferred embodiments for concurrent acquisition of harmonic and fundamental images for screening applications (which are intended to be illustrative and not limiting), it is noted that modifications and variations can be made by persons skilled in the art in light of the above teachings. It is therefore to be understood that changes may be made in the particular embodiments of the disclosure disclosed which are within the scope of the embodiments disclosed herein as outlined by the appended claims.

## Claims

1. A method for providing multiple review modes in a single acquisition scan, comprising:
acquiring (204) image frames for a plurality of ultrasound imaging modes including at least a fundamental mode and a harmonic mode by switching between ultrasound image acquisition modes including at least a fundamental acquisition mode and a harmonic acquisition mode in real-time during a single acquisition scan;
storing (208) the image frames in non-transitory memory for each of the ultrasound image acquisition modes for subsequent review; and
during review, selectively generating (210) a display for each of the plurality of ultrasound imaging modes from the stored image frames for an imaging mode selected by a reviewer such that each of the plurality of ultrasound imaging modes is available for review from the single acquisition scan,
wherein the method further comprises enabling or disabling (212) compounding using the stored images.

2. The method as recited in claim 1, further comprising selecting (202) a relative frame rate for each of the plurality of ultrasound imaging modes in the single acquisition scan.

3. The method as recited in claim 1, wherein acquiring (204) imaging frames includes acquiring imaging frames (206) from two imaging modes and the ultrasound image acquisition modes are switched such that frames are acquired in an alternating manner for each of the two imaging modes.

4. The method as recited in claim 1, wherein acquiring image frames (204) includes displaying a single imaging mode during the single acquisition scan.

5. The method as recited in claim 1, wherein the plurality of ultrasound imaging modes includes a first imaging mode for discovering first diagnostic information and a second imaging mode for discovering second diagnostic information for identifying a lesion in an

6. A non-transitory computer readable storage medium comprising a computer readable program for providing multiple review modes in a single acquisition scan, wherein the computer readable program when executed on a computer causes the computer to perform the steps of claim 1.

7. A system for providing multiple review modes in a single acquisition scan, comprising:
an ultrasound imaging device (10) configured to acquire image frames for a plurality of ultrasound imaging modes including at least a fundamental mode and a harmonic mode by automated switching between ultrasound image acquisition modes including at least a fundamental acquisition mode and a harmonic acquisition mode during a single acquisition scan;
a memory device (116) configured to store the image frames in non-transitory memory for each of the ultrasound image acquisition modes for subsequent review;
a signal processor (26) adapted to perform signal compounding; and
a review workstation (112) having a display (118) for viewing one of the plurality of ultrasound imaging modes from the stored image frames for an imaging mode selected by a reviewer such that each of the plurality of ultrasound imaging modes is available for review from the single acquisition scan, wherein compounding using the stored images can be enabled and disabled.

8. The system as recited in claim 7, further comprising a relative frame rate control (48) for setting a number of sequential frames for each of the plurality of ultrasound imaging modes in the single acquisition scan.

9. The system as recited in claim 8, wherein the relative frame rate includes an integer multiple of successive frames for at least one of the plurality of ultrasound imaging modes in the single acquisition scan.

10. The system as recited in claim 7, wherein the plurality of ultrasound imaging modes includes two imaging modes and the imaging modes are switched such that frames are acquired in an alternating manner for each of the two imaging modes.

11. The system as recited in claim 7, wherein the plurality of ultrasound imaging modes further include one or more of a compound fundamental mode, a compound harmonic mode, a color mode, a color power angioplasty mode and/or an elastography mode.

12. The system as recited in claim 7, wherein the review workstation (112) is configured for breast cancer screening.

## Patentansprüche

1. Verfahren zum Bereitstellen mehrerer Überprüfungsmodi in einem einzigen Erfassungsscan, umfassend:
Erfassen (204) von Bildframes für eine Vielzahl von Ultraschall-Bildgebungsmodi, einschließlich mindestens eines Grundmodus und eines harmonischen Modus, durch Umschalten zwischen Ultraschallbilderfassungsmodi, einschließlich mindestens eines Grunderfassungsmodus und eines harmonischen Erfassungsmodus, in Echtzeit während eines einzelnen Erfassungsscans;
Speichern (208) der Bildframes in einem nicht-transitorischen Speicher für jeden der Ultraschallbilderfassungsmodi zur späteren Überprüfung; und
beim Überprüfen, selektives Erzeugen (210) einer Anzeige für jeden der Vielzahl von Ultraschall-Bildgebungsmodi aus den gespeicherten Bildframes für einen Bildgebungsmodus, der von einem Überprüfer ausgewählt wurde, sodass jeder der Vielzahl von Ultraschall-Bildgebungsmodi für die Überprüfung aus dem einzelnen Erfassungsscan verfügbar ist,
wobei das Verfahren ferner ein Aktivieren oder Deaktivieren (212) der Zusammenstellung unter Verwendung der gespeicherten Bilder umfasst.

2. Verfahren nach Anspruch 1, ferner umfassend ein Auswählen (202) einer relativen Framerate für jeden der Vielzahl von Ultraschall-Bildgebungsmodi in dem einzelnen Erfassungsscan.

3. Verfahren nach Anspruch 1, wobei ein Erfassen (204) von Bildgebungsframes ein Erfassen von Bildgebungsframes (206) aus zwei Bildgebungsmodi umfasst und die Ultraschall-Bilderfassungsmodi geschaltet werden, sodass Frames abwechselnd für jeden der zwei Bildgebungsmodi erfasst werden.

4. Verfahren nach Anspruch 1, wobei ein Erfassen von Bildframes (204) ein Anzeigen eines einzelnen Bildgebungsmodus während des einzelnen Erfassungsscans umfasst.

5. Verfahren nach Anspruch 1, wobei die Vielzahl von Ultraschall-Bildgebungsmodi einen ersten Bildgebungsmodus zum Entdecken erster diagnostischer Informationen und einen zweiten Bildgebungsmodus zum Entdecken von zweiten diagnostischen Informationen zum Identifizieren einer Läsion in einem Organ (210) beinhaltet.

6. Nicht-transitorisches, computerlesbares Speichermedium, umfassend ein computerlesbares Programm zum Bereitstellen mehrerer Überprüfungsmodi in einem einzelnen Erfassungsscan, wobei das computerlesbare Programm, wenn es auf einem Computer ausgeführt wird, den Computer veranlasst, die Schritte nach Anspruch 1 auszuführen.

7. System zum Bereitstellen mehrerer Überprüfungsmodi in einem einzigen Erfassungsscan, umfassend:
eine Ultraschall-Bildgebungsvorrichtung (10), die konfiguriert ist, um Bildframes für eine Vielzahl von Ultraschall-Bildgebungsmodi, einschließlich mindestens eines Grundmodus und eines harmonischen Modus, durch Umschalten zwischen Ultraschallbilderfassungsmodi, einschließlich mindestens eines Grunderfassungsmodus und eines harmonischen Erfassungsmodus, in Echtzeit während eines einzelnen Erfassungsscans zu erfassen;
eine Speichervorrichtung (116), die konfiguriert ist, um die Bildframes in einem nicht-transitorischen Speicher für jeden der Ultraschallbilderfassungsmodi zur späteren Überprüfung zu speichern;
einen Signalprozessor (26), der angepasst ist, um eine Signalaufbereitung auszuführen; und
eine Überprüfungs-Workstation (112), die eine Anzeige (118) zum Betrachten eines der Vielzahl von Ultraschall-Bildgebungsmodi aus den gespeicherten Bildframes für einen Bildgebungsmodus aufweist, der von einem Überprüfer ausgewählt wird, sodass jeder der Vielzahl von Ultraschall-Bildgebungsmodi zum Überprüfen aus dem einzelnen Erfassungsscan verfügbar ist, wobei das Zusammensetzen unter Verwendung der gespeicherten Bilder aktiviert und deaktiviert werden kann.

8. System nach Anspruch 7, ferner umfassend eine relative Frameratensteuerung (48) zum Einstellen einer Anzahl aufeinanderfolgender Frames für jeden der Vielzahl von Ultraschall-Bildgebungsmodi in dem einzelnen Erfassungsscan.

9. System nach Anspruch 8, wobei die relative Framerate ein ganzzahliges Vielfaches aufeinanderfolgender Frames für mindestens einen der Vielzahl von Ultraschall-Bildgebungsmodi in dem einzelnen Erfassungsscan umfasst.

10. System nach Anspruch 7, wobei die Vielzahl von Ultraschall-Bildgebungsmodi zwei Bildgebungsmodi umfasst und die Bildgebungsmodi geschaltet sind, sodass Frames abwechselnd für jeden der zwei Bildgebungsmodi erfasst werden.

11. System nach Anspruch 7, wobei die Vielzahl von Ultraschall-Bildgebungsmodi ferner einen oder mehrere von einem zusammengesetzten Grundmodus, einem zusammengesetzten harmonischen Modus, einem Farbmodus, einem Farb-Power-Angioplastie-Modus und/oder einem Elastographie-Modus umfasst.

12. System nach Anspruch 7, wobei die Überprüfungs-Workstation (112) für Brustkrebs-Screening konfiguriert ist.

## Revendications

1. Procédé pour fournir plusieurs modes d'examen dans un balayage d'acquisition individuel, consistant à:
acquérir (204) des trames d'image pour une pluralité de modes d'imagerie par ultrasons incluant au moins un mode fondamental et un mode harmonique en commutant entre les modes d'acquisition d'image par ultrasons incluant au moins un mode d'acquisition fondamental et un mode d'acquisition harmonique en temps réel au cours d'un balayage d'acquisition individuel;
stocker (208) les trames d'image dans une mémoire non transitoire pour chacun des modes d'acquisition d'image par ultrasons pour examen ultérieur; et
pendant l'examen, générer sélectivement (210) un affichage pour chacun de la pluralité de modes d'imagerie par ultrasons à partir des trames d'image stockées pour un mode d'imagerie sélectionné par un examinateur de sorte que chacun de la pluralité de modes d'imagerie par ultrasons est disponible pour examen à partir du balayage d'acquisition individuel,
dans lequel le procédé consiste en outre à activer ou désactiver (212) la composition à l'aide des images stockées.

2. Procédé selon la revendication 1, consistant en outre à sélectionner (202) une fréquence de trame relative pour chacun de la pluralité de modes d'imagerie par ultrasons dans le balayage d'acquisition individuel.

3. Procédé selon la revendication 1, dans lequel l'acquisition (204) de trames d'imagerie inclut l'acquisition de trames d'imagerie (206) à partir de deux modes d'imagerie et les modes d'acquisition d'image par ultrasons sont commutés de sorte que les trames sont acquises d'une manière alternée pour chacun des deux modes d'imagerie.

4. Procédé selon la revendication 1, dans lequel l'acquisition de trames d'image (204) inclut l'affichage d'un mode d'imagerie individuel pendant le balayage d'acquisition individuel.

5. Procédé selon la revendication 1, dans lequel la pluralité de modes d'imagerie par ultrasons inclut un premier mode d'imagerie pour découvrir de premières informations de diagnostic et un second mode d'imagerie pour découvrir de secondes informations de diagnostic pour identifier une lésion dans un organe (210).

6. Support de stockage lisible par ordinateur non transitoire comprenant un programme lisible par ordinateur pour fournir de multiples modes d'examen dans un balayage d'acquisition individuel, dans lequel le programme lisible par ordinateur, lorsqu'il est exécuté sur un ordinateur, amène l'ordinateur à mettre en œuvre les étapes de la revendication 1.

7. Système pour fournir plusieurs modes d'examen dans un balayage d'acquisition individuel, comprenant:
un dispositif d'imagerie par ultrasons (10) configuré pour acquérir des trames d'image pour une pluralité de modes d'imagerie par ultrasons incluant au moins un mode fondamental et un mode harmonique en commutant automatiquement entre les modes d'acquisition d'image par ultrasons incluant au moins un mode d'acquisition fondamental et un mode d'acquisition harmonique au cours d'un balayage d'acquisition individuel;
un dispositif de mémoire (116) configuré pour stocker les trames d'image dans une mémoire non transitoire pour chacun des modes d'acquisition d'image par ultrasons pour examen ultérieur;
un processeur de signal (26) adapté pour effectuer une composition de signal; et
une station de travail d'examen (112) présentant un affichage (118) pour visualiser un de la pluralité de modes d'imagerie par ultrasons à partir des trames d'image stockées pour un mode d'imagerie sélectionné par un examinateur de sorte que chacun de la pluralité de modes d'imagerie par ultrasons est disponible pour examen à partir du balayage d'acquisition individuel, dans lequel une composition utilisant les images stockées peut être activée et désactivée.

8. Système selon la revendication 7, comprenant en outre une commande de fréquence de trame relative (48) pour régler un certain nombre de trames séquentielles pour chacun de la pluralité de modes d'imagerie par ultrasons dans le balayage d'acquisition individuel.

9. Système selon la revendication 8, dans lequel la fréquence de trame relative inclut un multiple entier de trames successives pour au moins un de la pluralité de modes d'imagerie par ultrasons dans le balayage d'acquisition individuel.

10. Système selon la revendication 7, dans lequel la pluralité de modes d'imagerie par ultrasons inclut deux modes d'imagerie et les modes d'imagerie sont commutés de sorte que les trames sont acquises d'une manière alternée pour chacun des deux modes d'imagerie.

11. Système selon la revendication 7, dans lequel la pluralité de modes d'imagerie par ultrasons inclut en outre un ou plusieurs parmi un mode fondamental composé, un mode harmonique composé, un mode couleur, un mode angioplastie à puissance de couleur et/ou un mode élastographie.

12. Système selon la revendication 7, dans lequel la station de travail d'examen (112) est configurée pour le dépistage du cancer du sein.
